# EUROPEAN PATENT SPECIFICATION

(11) **EP 3 715 447 B1**
(45) Date of publication and mention of the grant of the patent: **16.03.2022**
(21) Application number: 20165727.7
(22) Date of filing: 25.03.2020
(51) Int. Cl.: C12M 1/32, C12M 1/12, C12M 1/42, C12M 3/06

(54) **CELL CULTURE DEVICE AND ELECTROMAGNETIC STIMULATION CELL CULTURING SYSTEM HAVING THE SAME**
ZELLKULTURVORRICHTUNG UND ELEKTROMAGNETISCHES STIMULATIONSZELLKULTURSYSTEM DAMIT
DISPOSITIF DE CULTURE CELLULAIRE ET SYSTÈME DE CULTURE CELLULAIRE À STIMULATION ÉLECTROMAGNÉTIQUE LE COMPORTANT

(30) Priority: 28.03.2019 TW 108111009
(43) Date of publication of application: 30.09.2020
(73) Proprietor: National Health Research Institutes, Miaoli County 35053 (TW)
(72) Inventor: LIN, FENG-HUEI, 350 Miaoli County (TW); HUANG, SHU-WEI, 350 Miaoli County (TW); TZENG, SHIAN-CHIUAN, 350 Miaoli County (TW); TSENG, KUO-HSUN, 333 Taoyuan City (TW)
(74) Representative: Lang, Christian

(56) References cited:
- EP-A1- 2 811 012
- WO-A2-2011/094572
- US-A1- 2013 084 634
- US-A1- 2016 102 281

## Description

### BACKGROUND OF THE INVENTION

### Field of the Invention

The present invention relates to a cell culture device and electromagnetic stimulation cell culturing system having the same, and in particular to a suspension type cell culture device and electromagnetic stimulation cell culturing system having the same.

### The Prior Arts

In the past several decades, the research of cell biology has raised our understanding of quite a lot of basic biological problems, and it has promoted the research of cell treatment methods for various diseases. The research of the cells is aimed at obtaining an optimal cell model, and its unlimited proliferation potential and multi-curing capabilities lead to the growth, differentiation, and development of cells for mammal especially human. Since the proliferation and differentiation of vivo cells and vitro cells can be regulated by specific signals, so we can simulate this type of cells in the body, such that it has great value and potential in clinical treatment. However, it is rather difficult to cultivate large quantity of cells outside the body.

In the past, 2D culture was used to produce massive proliferation of cells. Yet the cell produced in this approach tends to proliferate in an unpredictable manner, and it is not able to keep the shape of the cell prototype. Therefore, the cell culture turns to 3D culture. In the early stage, bioreactor is used in 3D culture. In general, that requires to use an artificial stereo biological scaffold, to make the cells attach to the surface of the scaffold to achieve cultivation. However, the artificial stereo biological scaffold is not able to simulate the genuine environment in the human body. Besides, presently, the biological scaffold is formed by chemical materials, and it is liable to induce cell differentiation.

Presently, the bioreactor cell cultivation is realized through using a tissue culturing system, in which, the single cell and small cell mass are cultured and obtained in a liquid culture medium by stirring and shaking continuously and incessantly the liquid culture medium. The liquid culture medium is a cultivar of non-adherent cell, the cell aggregate thus produced must be attached onto a biological culture medium to cultivate. However, the biological culture mediums are mostly formed by synthetic chemical substances, and is liable to induce differentiation of cells. As such, it is not able to simulate the cultivation environment for a cell in a biological body. In this respect, the cell suspension culture is a way to produce large quantity of proliferated cells. Namely, in a fresh liquid culture medium, use the gravitation force and the aggregation force of water to simulate the stereo growth environment for the cells in the body, to cultivate the cells in a stationary condition. Then repeat the subculture process continuously, to obtain large quantity of suspended cell aggregates in a short time. However, the technique of suspension cultivation is rather difficult, since it depends on the friction force between the cell culture plate and the water drop to hold and fix the water drop. When subjected to shaking or vibration, the water drops are certain to fall, to cause death of cells. Moreover, the cell culture liquid in the suspension type culture plate can not be replenished automatically, thus long time cell cultivation is not possible. Therefore, presently, there is an urgent need to have a suspension cell cultivation device, for which the culture liquid can be replenished regularly and stably, to cultivate large quantity of cell aggregates having good and stable quality.

Further, according to a large number of Documents, when the cell in cultivation is subjected to electromagnetic stimulations, that is helpful to its proliferation and variations of morphology. The behavior of the cell (migration, differentiation, and repair) is related to the voltage gradient produced by vivo and vitro environments of the cell. The current thus produced is caused by the sodium ions and chlorine ions in vitro the cell, and the potassium ions in vivo the cell. The cell membrane is able to control selectively the passage of ions, to cause the imbalance of ion flow between the in vivo cell and the in vitro cell, and to cause charge polarization of the cell membrane, hereby producing membrane potential. In the human body, the variation of inside electromagnetic field is evident, that for example could be caused by the muscle and bone operations, such that voltage is induced by variations of the potentials. Currently, the research results indicate that, through the influence of the outside electromagnetic field, the proliferation and differentiation of the cells can be regulated effectively, similar to regulation of the properties of the medicine molecules through the outside magnetic field.

Microfluidic devices for cell culturing using hanging droplets are known from US 2013/084634 A1, WO 2011/094572 A2, US 2016/102281 A1 and EP 2811012 A1.

Therefore, at present, there is an urgent need to have an electromagnetic stimulation cell culturing device capable of regulating the cell culture medium automatically, to raise the efficiency of cell cultivation, and to cultivate large quantity of cells having stable and reliable quality.

### SUMMARY OF THE INVENTION

In view of the problems and drawbacks of the Prior Art, the present invention provides a cell culture device and an electromagnetic stimulation cell culturing system having the same, to overcome the shortcomings of the Prior Art. The cell culture device is adapted to suspend the cell culture liquid under the suspension unit by means of suspension, and it utilizes flow circulation to replace and replenish the cell culture liquid. In addition, the suspension hole is of upper part narrow and lower part wide shape, to match the shape of liquid drops, and its cross section is in a wedge shape of narrow upper part and wide lower part. In this way, the shape of the suspension hole matches that of suspended liquid drop, to suspend the liquid drops stably under the suspension unit. Moreover, in the present invention, the cell culture device can be used in the electromagnetic stimulation cell culturing system. And the electromagnetic stimulation cell culturing system includes a cell culture device, a flow body supply unit, and an electromagnetic field generation unit. As such, the electromagnetic stimulation cell culturing system is a combination of hanging drop method, microfluidic technology, and electromagnetic stimulation cell culturing technology, to form a cell culturing system capable of providing continuously cell culture liquid into the cell culture device. Therefore, the cell culture device and the electromagnetic stimulation cell culturing system of the present invention is capable of raising the efficiency of cell culturing, in achieving cultivation of large quantity of cells of stable and reliable quality.

In order to achieve the objective mentioned above, the present invention provides a cell culture device, that includes a top layer, a diffusion layer, and a suspension layer. The top layer is provided with at least an inlet hole and at least an outlet hole, the inlet hole is used to input a flow body into the cell culture device, the outlet hole is used to output the flow body from the cell culture device. The diffusion layer is provided with at least a flow channel, the flow channel corresponds to the at least an inlet hole and the at least an outlet hole, and is used to guide the flow body. The suspension layer is provided with a plurality of suspension units, the suspension units correspond to the flow channel. The flow body flows into the suspension units through the flow channel, and is suspended under the suspension units, so that the cells are cultured in the flow body suspended under the suspension units respectively. Wherein, each of the suspension units is provided with a suspension hole and a protrusion structure, and the suspension hole runs through the suspension layer, and having an upper hole and a lower hole. The diameter of the upper hole is less than the diameter of the lower hole. The flow body flows into the suspension hole from the upper hole, the protrusion structure is extended and disposed below the lower hole, so that the flow body is suspended under the suspension units along the protrusion structure.

The present invention also provides an electromagnetic stimulation cell culturing system used to culture cells. The culturing system utilizes a suspension approach to hang the cell culture liquid under the suspension unit, and it utilizes an outside electromagnetic field to induce proliferation of cells. The electromagnetic stimulation cell culturing system is capable of raising the efficiency of cell culturing, to produce large quantity of cells having reliable and stable quality.

In order to achieve the objective mentioned above, the present invention provides an electromagnetic stimulation cell culturing system to culture cells, and it includes: a cell culture device, a receiving plate, a flow body supply unit, and an electromagnetic field generation unit. The receiving plate is disposed below the cell culture device, and having a bottom seat and a plurality of receiving slots. The plurality of receiving slots correspond to the plurality of the suspension units of the cell culture device respectively, and are used to receive and collect the cells and the flow body. The flow body supply unit is connected to the cell culture device, and is used to supply the flow body in circulation. The electromagnetic field generation unit is adapted to generate an electromagnetic field around the cell culture device.

Summing up the above, the present invention provides a cell culture device and electromagnetic stimulation cell culturing system having the same, that is adapted to suspend the cell culture liquid under the suspension units by means of suspension, and it utilizes flow circulation to replace and replenish the cell culture liquid. An outside electromagnetic field is used to induce growth of the cells.

In addition, the suspension hole is of upper part narrow and lower part wide shape, matching the shape of liquid drops, and its cross section is of a wedge shape having upper side narrow and lower side wide. In this way, the shape of the suspension hole matches the shape of suspended liquid drop having its upper part narrow and its lower part wide, so it can be suspended stably under the suspension unit. Moreover, in the present invention, the cell culture device can be applied in the electromagnetic stimulation cell culturing system. And the electromagnetic stimulation cell culturing system includes a cell culture device, a flow body supply unit, and an electromagnetic field generation unit. As such, the electromagnetic stimulation cell culturing system combines hanging drop method, microfluidic technology, and electromagnetic field stimulation cell culturing technology, to form a cell culturing system capable of supplying cell culture liquid stably and continuously. Therefore, the cell culture device and the electromagnetic stimulation cell culturing system of the present invention are capable of raising the efficiency of cell culturing, while culturing large quantity of cells having stable and reliable quality.

### BRIEF DESCRIPTION OF THE DRAWINGS

The related drawings in connection with the detailed descriptions of the present invention to be made later are described briefly as follows, in which:
Fig. 1 is an exploded view of a cell culture device according to the first embodiment of the present invention;
Fig. 2 is an exploded view of a cell culture device according to the second embodiment of the present invention;
Fig. 3A is a cross section view of cell culture device along L1-L1 line of Fig. 1 according to the first embodiment of the present invention;
Fig. 3B is a perspective cross section view of cell culture device along L1-L1 line of Fig. 1 according to the first embodiment of the present invention;
Fig. 4A is a partial enlarged view of the suspension unit of the cell culture device in Fig. 3A according to the present invention;
Fig. 4B is a partial enlarged perspective view of the suspension unit of the cell culture device in Fig. 3B according to the present invention;
Fig. 4C is a schematic diagram of the cell culture liquid and the culture cells suspended under the suspension unit of Fig. 4A according to the present invention;
Fig. 5A is a perspective view of the receiving plate of the electromagnetic stimulation cell culturing system according to the present invention;
Fig. 5B is a perspective view of the receiving plate of the electromagnetic stimulation cell culturing system viewed from another angle according to the present invention;
Fig. 5C is a cross section view of the receiving plate of the electromagnetic stimulation cell culturing system along the L2-L2 line of Fig. 5A according to the present invention;
Fig. 6A is a schematic diagram of the electromagnetic stimulation cell culturing system according to the third embodiment of the present invention; and
Fig. 6B is a schematic diagram of the electromagnetic stimulation cell culturing system according to the fourth embodiment of the present invention.

### DETAILED DESCRIPTION OF THE PREFERRED EMBODIMENT

The purpose, construction, features, functions and advantages of the present invention can be appreciated and understood more thoroughly through the following detailed descriptions with reference to the attached drawings.

In the following, an embodiment is used to describe the various details of the present invention. However, it does not mean that this embodiment represents all the embodiments of the present invention. Other embodiments can be envisaged by people familiar with this field, and thus they all fall into the scope of the present invention as defined by the claims.

The electromagnetic stimulation cell culturing system of the present invention is a combination of hanging drop method, microfluidic technology, and electromagnetic field stimulation cell culturing technology, to form a cell culturing system capable of providing continuously culture medium into the cell culture device.

The hanging drop method refers to a technology of cultivating cells in the suspended culture liquid drops.

The Microfluidic Technology is used to perform precision cutting for material of minute size, to form micro channels having liquid inlet ends and liquid outlet ends. Then it utilizes a micro pump to push the liquid and transport it through the micro channel.

The electromagnetic field stimulation cell culturing technology relates to a technology concerning electromagnetic field, and it is characterized by the following parameters: current intensity of AC and DC, the current in unit ampere (A), the voltage in unit voltage (V), magnetic flux density in unit of Gauss (G) or Tesla (T). The electromagnetic field strength can be calculated by making use of Faraday's law of electromagnetic induction and Maxwell Equation. In this respect, a charged particle usually moves with a constant velocity, and when it is accelerated, it will generate an electric field and a magnetic field in combination at the same time, thus producing an electromagnetic field. The electrical field and the magnetic field influence each other to produce oscillations. The higher the oscillation frequency, the more intense the coupling of the magnetic field and the electrical field.

In the present invention, the cell culture device can be used in the electromagnetic stimulation cell culturing system. And the electromagnetic stimulation cell culturing system includes a cell culture device, a flow body supply unit, and an electromagnetic field generation unit. As such, the electromagnetic stimulation cell culturing system is a combination of hanging drop method, a microfluidic technology, and electromagnetic field stimulation cell culturing technology, to form a cell culturing system capable of providing culture medium continuously into the cell culture device. Therefore, the cell culture device and the electromagnetic stimulation cell culturing system of the present invention are capable of raising the efficiency of cell culturing, to culture large quantity of cells of stable and reliable quality.

In the following, a preferred embodiment of the present invention is described in detail to explain and illustrate various details of the present invention.

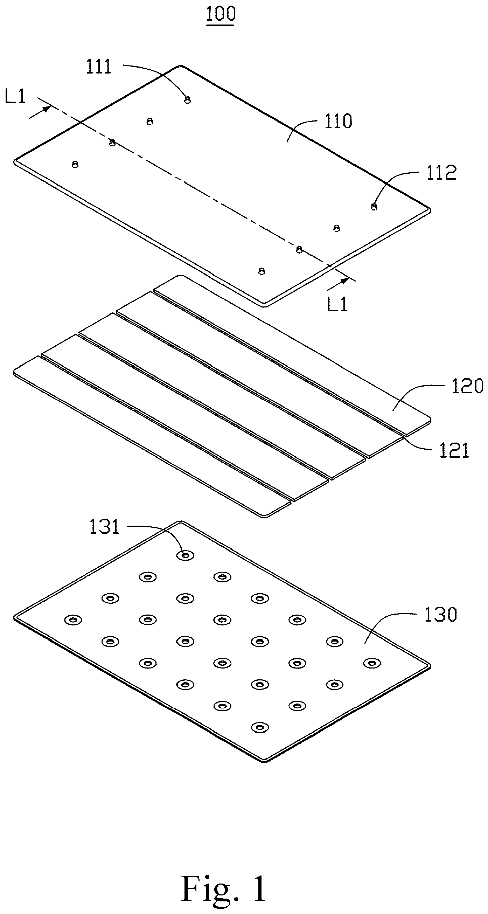

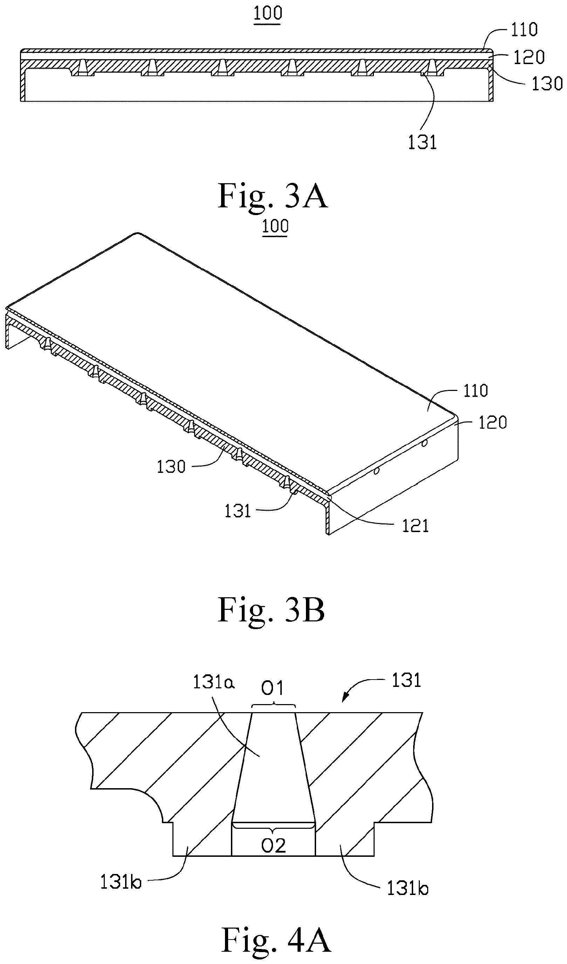

Refer to Figs. 1, 3A, 3B respectively for an exploded view of a cell culture device according to a first embodiment of the present invention; a cross section view of cell culture device along L1-L1 line of Fig. 1 according to the first embodiment of the present invention; and a perspective cross section view of cell culture device along L1-L1 of Fig. 1line according to the first embodiment of the present invention. As shown in Fig. 1, the cell culture device 100 includes from top to bottom a top layer 110, a diffusion layer 120, and a suspension layer 130. The top layer 110 is provided with at least an inlet hole 111 and at least an outlet hole 112. The inlet hole 111 is used to allow a flow body, such as cell culture liquid into the cell culture device 100. The cell culture liquid can be for example a Dulbecco's Modified Eagle's Medium Sigma-Aldrich (DMEM) cell culture liquid. The water outlet hole 112 is used to output the flow body out of the cell culture device 100.

As shown in Fig. 1, in the first embodiment, the top layer 110 is provided with four inlet holes 111, and four corresponding outlet holes 112. In the diffusion layer 120 is disposed at least a flow channel 121. The flow channel 112 corresponds to the at least an inlet hole 111, and the at least an outlet hole 112, and is used to allow the flow body to flow through. In the first embodiment, the four inlet holes 111 of the top layer 110 and the four outlet holes 112 of the top layer 110 correspond respectively to the four flow channels 121 of the diffusion layer 120. As such, the flow body flows in the flow channel 121 through the inlet hole 111, and then it flows out from the outlet hole 112. The suspension layer 130 is provided with a plurality of suspension units 131, and the plurality of suspension units 131 correspond to the flow channel 121. The flow body flows into the suspension units 131 through the flow channels 121, and is suspended under the suspension units 131, so that cells are cultured in the flow body under the suspension unit 131.

As shown in Fig.1, in the first embodiment, each flow channel 121 correspond to six suspension units 131. In other words, each flow channel 121 is disposed to guide the flow body into six suspension units 131. As shown in Fig. 1, in the suspension layer 130, 24 suspension units 131 are provided, that can be used to culture 24 groups of cells.

As shown in Figs. 3A and 3B, the top layer 110, the diffusion layer 120, and the suspension layer 130 are formed integrally into a body. The top layer 110, the diffusion layer 120, and the suspension layer 130 can be made of Acrylic or Polydimethylsiloxane (PDMS). The top layer 110, the diffusion layer 120, and the suspension layer 130 can first be made separately, then put together into a body. The thickness of the top layer 110 is 1 to 10 mm, the thickness of the diffusion layer 120 is 1 to 10 mm, and the thickness of the suspension layer 130 is 1 to 20 mm.

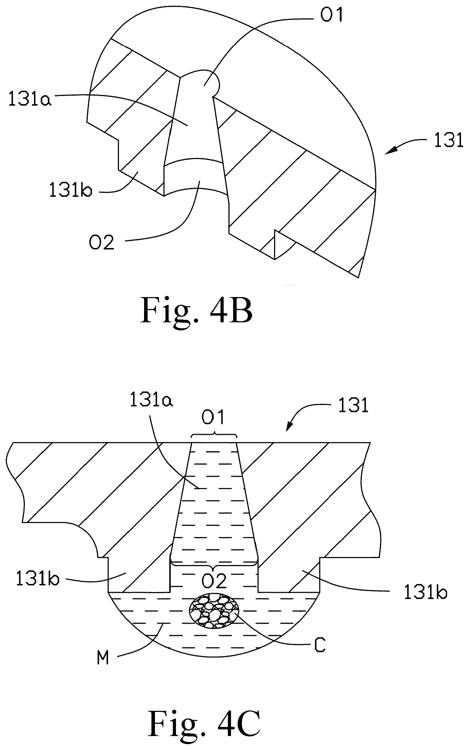

Refer to Figs. 4A, 4B, and 4C respectively for a partial enlarged view of the suspension unit of the cell culture device in Fig. 3A according to the present invention; a partial enlarged perspective view of the suspension unit of the cell culture device in Fig. 3B according to the present invention; and a schematic diagram of the culture liquid and the culture cell suspended under the suspension unit of Fig. 4A according to the present invention.

As shown in Figs. 4A, 4B, the suspension unit 131 includes a suspension hole 131a and a protrusion structure 131b. The suspension hole 131a runs through the suspension layer 100, and having an upper hole 01 and a lower hole 02. The diameter of the upper hole 01 is less than the diameter of the lower hole 02. Therefore, the suspension hole 131a is of an upper part narrow and lower part wide shape, with its cross section in a wedge shape of upper part narrow and lower part wide. The flow body flows into the suspension hole 131a through the upper hole 01, the protrusion structure 131b extends below the lower hole 02. The flow body is suspended under the suspension unit 131 along the protrusion structure 131b. The diameter of the upper hole 01 is 1-15mm, while the diameter of the lower hole 02 is more than 1 mm to 30 mm, and the height of the protrusion structure 131b is 1 to 20mm.

As shown in Fig. 4C, the flow body is an ordinary liquid, such as a cell culture liquid, that can be suspended below the lower hole 02 by means of surface tension. The upper part narrow and lower part wide structure of the suspension hole 131a is just fit for the shape of the liquid drop, hereby increasing the contact area between the flow body and the suspension hole 131a. As such, the flow body is suspended stably in the suspension hole 131a and will not drop down. The protrusion structure 131b is of a ring shape structure, extended and disposed below the lower hole 02. Likewise, the protrusion structure 131b is fit for the shape of liquid drop, thus increasing the overall contact area between the flow body and the suspension unit 131. As such, the flow body is suspended stably in the suspension hole 131a and will not drop down. In this way, the cell C is cultured in the flow body (in the liquid drop of the cell culture liquid) located in the suspension unit 131 in a suspension way. As mentioned earlier, fresh cell culture liquid may flow into the flow channel 121 via the inlet hole 111, and then it flows into the suspension unit 131, to form liquid drop suspended under the suspension unit 131, to perform cell cultivation. Finally, the flow body flows through the flow channel 121, and then flows out of the cell culture device 100 from the outlet hole 112. In this way, the cell culture device 100 is able to provide continuously fresh cell culture liquid to perform cultivation of the Cell C, and expel excrements produced in the cell culturing process.

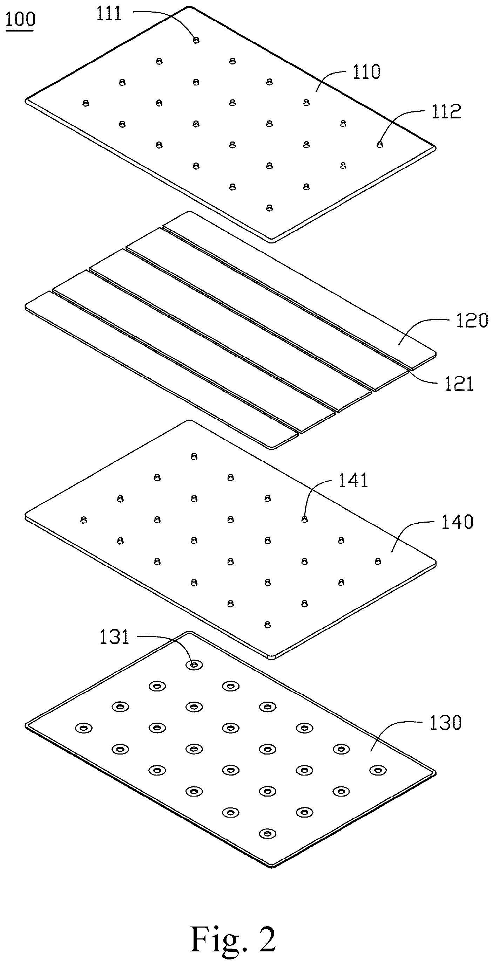

Refer to Fig. 2 for an exploded view of a cell culture device according to a first embodiment of the present invention. As shown in Fig. 2, the difference between the cell culture device 100 of the second embodiment in Fig. 2, and the cell culture device 100 of the first embodiment in Fig. 1 is that, in Fig. 2, in addition to the top layer 110, the diffusion layer 120, and the suspension layer 130, a separation layer 140 is provided. The separation layer 140 is disposed between the diffusion layer 120 and the suspension layer 130. The separation layer 140 is provided with a plurality of separation holes 141 corresponding to the suspension units 131. The flow body flows into the upper hole 01 of the separation holes 131a through the flow channels 121 and the separation holes 141. As shown in Fig. 2, in the second embodiment, the separation layer 140 is provided with 24 separation holes 141 corresponding to the 24 suspension units 131.

The diameter of the separation hole 141 is less than that of the upper hole 01. In the flow channel 121, the flow body first flows into the separation holes 141 and then it flows into the suspension unit 131 corresponding to the separation hole 141. In this way, it can avoid the flow body in the flow channel 121 from flowing directly into the suspension unit 131. Instead, the flow body first flows into the separation hole 141 of smaller diameter, so that the flow body may be suspended under the suspension unit 131 more stably. As such, it could prevent dropping down of the liquid drops of the cell culture liquid from the suspension unit 131, due to disturbance of the flow body in the flow channel. In the second embodiment, the structures and materials of the top layer 110, the diffusion layer 120, and the suspension layer 130 utilized are the same as those of the first embodiment.

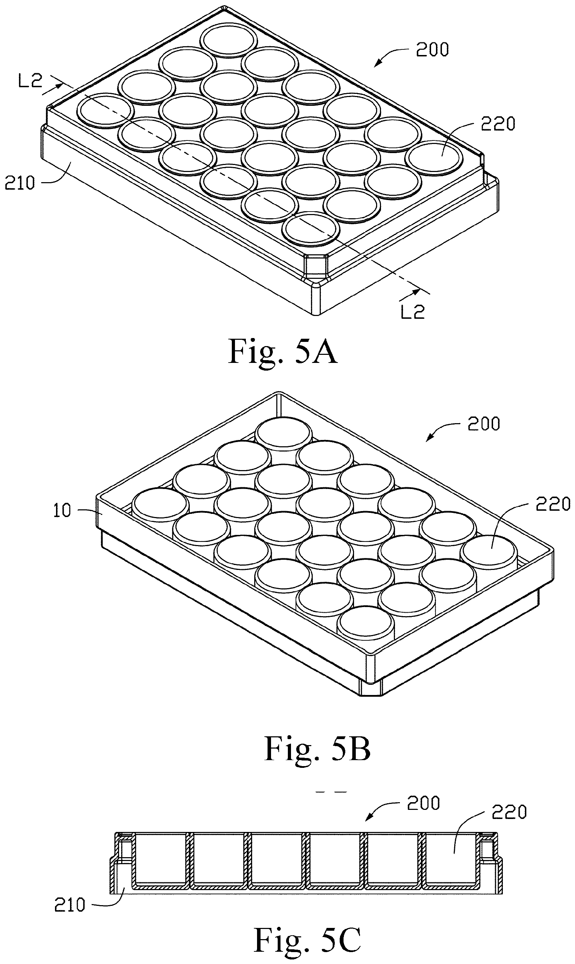

In application, the cell culture device 100 is placed above the receiving plate, and upon completion of cell culturing, the cell culture device 100 can be knocked or shaken slightly, to cause the cell culture liquid and the cells thus formed to fall down onto the receiving plate. In the present invention, by utilizing the receiving plate 200, the cell culture device 100 can be used in the electromagnetic simulation cell culturing system to perform cell culturing. Refer to Figs. 5A, 5B, and 5C respectively for a perspective view of the receiving plate of the electromagnetic stimulation cell culturing system according to the present invention; a perspective view of the receiving plate of the electromagnetic stimulation cell culturing system viewed from another angle according to the present invention; and a cross section view of the receiving plate of the electromagnetic stimulation cell culturing system along the L2-L2 line of Fig. 5A according to the present invention. As shown in Figs. 5A, 5B, and 5C, the receiving plate 200 is provided with a bottom seat 210, and plurality of receiving slots 220 on the bottom seat 210. The plurality of receiving slots 220 correspond to the plurality of the suspension units 131 of the cell culture device 100, to receive the flow body and the cells cultured. Figs. 5A and 5B, the receiving plate 200 is provided with 24 receiving slot 220 corresponding to the 24 suspension unit 131 of the first and second embodiments. The receiving slots 220 can be of a cylinder shape indent slot, and are disposed on the bottom seat 210.

In culturing cells by using the cell culture device 100, an outside electromagnetic field can be applied to simulate the proliferation and differentiation of the cells. In general, in the growing process of a living body, an electric field is generated in the living body. And that plays an important role in embryo growth, healing of wounds, tissue repair, and normal growth and maturity. The electromagnetic field exists in the cytoplasm and the space outside the cell, and having its current intensity ranging from several mv/mm to several hundred mv/mm. In clinical application, current treatment is used extensively, and it is especially effective for increasing the healing speed of damaged neuro-muscles. Therefore, by placing a living body or cell in an electromagnetic field of a certain magnitude, physiological and biochemical reactions can be induced, to promote proliferation and differentiation of the cells. In the following, the application of the cell culture device 10 in the electromagnetic stimulation cell culturing system is described and explained in detail.

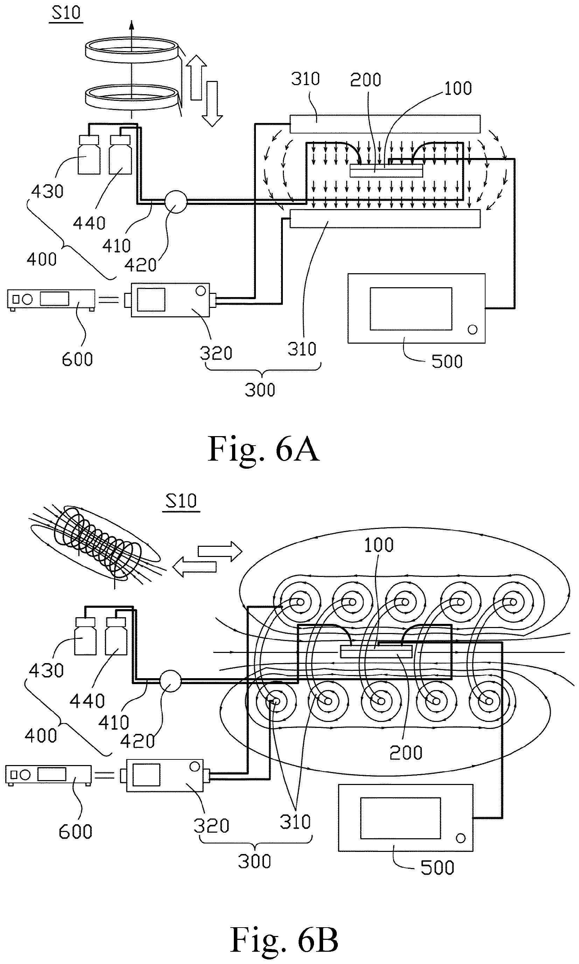

Refer to Fig. 6A for a schematic diagram of the electromagnetic stimulation cell culturing system according to the third embodiment of the present invention, As shown in Fig. 6A, the electromagnetic stimulation cell culturing system S10 includes a cell culture device 100, a receiving plate 200, a flow body supply unit 400, and an electromagnetic field generation unit 300. The receiving plate 200 is disposed below the cell culture device 100, the flow body supply unit 400 is connected to the cell culture device 100, and is used to provide the flow body in circulation. The electromagnetic field generation unit 300 generates an electromagnetic field around the cell culture device 100. The flow body supply unit 400 includes a pump 420, and a circulation channel 410. The circulation channel 410 is connected to the inlet hold 111 and the outlet hole 112 of the cell culture device 100, and is used to input or output the flow body; while the pump 420 is adapted to push the flow body to flow in circulation. The flow body supply unit 400 further includes a supply container 430 and a receiving container 440. The supply container 430 is connected to the inlet hole 111 of the cell culture device 100 through the circulation channel 410. The receiving container 440 is connected to the outlet hole 112 of the cell culture device 100 through the circulation channel 410. The electromagnetic field generation unit 300 includes at least a coil 310 and a magnetic field controller 320. The at least a coil 310 is adapted to generate an electromagnetic field. The magnetic field controller 320 is connected to the at least a coil 310, and is adapted to control the direction, frequency, and intensity of the electromagnetic field. In the present embodiment, the coil 310 is formed by two coils parallel to each other. The cell culture device 100 and the receiving plate 200 are placed between the two parallel coils 310, as such the magnetic field generated by the coil is perpendicular to that of the cell culture device 100. As shown in Fig. 6A, the electromagnetic stimulation cell culturing system S10 further includes a power supplier 600, and is used to supply power to the magnetic field controller 320. In addition, the electromagnetic stimulation cell culturing system S10 further includes a magnetic field detector 500, used to detect the electromagnetic field generated by the coil 310. In the present embodiment, the functions and structures of the cell culture device 100 and the receiving plate 200 are the same as those of the previous embodiments. In the present embodiment, the flow body supply unit 400, the electromagnetic field generation unit 300, the power supplier 600, and the magnetic field detector 50 all belong to the existing technology, their functions and structures are well known, thus that will not be described here in detail for brevity.

Refer to Fig. 6B for a schematic diagram of the electromagnetic stimulation cell culturing system according to the fourth embodiment of the present invention. The differences between the electromagnetic stimulation cell culturing systems S10 of the third embodiment and the fourth embodiment is that, in the present embodiment, as shown in Fig. 6B, the coil 310 is a spiral coil, and the cell culture device 100 and the receiving plate 200 are placed in the spiral coil. As such, the magnetic field generated by the spiral coil is parallel to that of the cell culture device 100.

As shown in Figs. 6A and 6B, magnetic fields of different magnetic directions (parallel or perpendicular) can be used to induce different effects for the cells, to produce different cultured cells. In addition to the direction of the magnetic field, the intensity of the magnetic field and the varying frequencies of the magnetic field, and the compositions of the cell culture liquid could affect the proliferations of the cells, to produce different cultured cells. Therefore, different cells can be cultured by changing the conditions mentioned above. The technology of cell culturing belongs to the Prior Art, and it will not be repeated here for brevity.

Summing up the above, in the present invention, the cell culture device and electromagnetic stimulation cell culturing system make use of the suspension approach to suspend the cell culture liquid under the suspension unit, so that cells are cultivated in the suspended cell culture liquid, and it utilizes flow circulation to replace and replenish the cell culture liquid. An electromagnetic field is applied from outside to induce proliferations of the cells. In addition, the suspension hole is of upper part narrow and lower part wide shape, with its cross section also in a wedge shape of upper part narrow and lower part wide. As such, the shape of the suspension hole match that of the liquid drop in suspension, in achieving stable suspension of the liquid drops under the suspension unit. Moreover, in the present invention, the cell culture device can be used in the electromagnetic stimulation cell culturing system, such that the electromagnetic stimulation cell culturing system includes a cell culture device, a flow body supply unit, and an electromagnetic field generation unit. As such, the electromagnetic stimulation cell culturing system is a combination of hanging drop method, a microfluidic technology, and electromagnetic stimulation cell culturing technology, to form a cell culture system capable of providing continuously and stably the cell culture liquid into the cell culture device. Therefore, the cell culture device and the electromagnetic stimulation cell culturing system of the present invention is capable of raising the efficiency of cell cultivation, in achieving cultivation of large quantity of cells of a stable and reliable quality.

The above detailed description of the preferred embodiment is intended to describe more clearly the characteristics of the present invention. However, the preferred embodiments disclosed above are not intended to be any restrictions to the scope of the present invention. Conversely, its purpose is to include the various changes and equivalent arrangements which are within the scope of the appended claims.

## Claims

1. A cell culture device (100) used to culture cells, comprising:
a top layer (110), having at least an inlet hole (111) and at least an outlet hole (112), the inlet hole (111) is used to input a flow body (M) into the cell culture device (100), the outlet hole (112) is used to output the flow body (M) from the cell culture device (100);
a diffusion layer (120), having at least a flow channel (121), the flow channel (121) corresponds to the inlet hole (111) and the outlet hole (112), and is used to guide the flow body (M); and
a suspension layer (130), having a plurality of suspension units (131), the suspension units (131) correspond to the flow channel (121), the flow body (M) flows into the suspension units (131) through the flow channel (121), and is suspended under the suspension units (131), so that the cells (C) are cultured in the flow body (M) under the suspension units (131) respectively, wherein, each of the suspension units (131) is provided with a suspension hole (131a) and a protrusion structure (131b), the suspension hole (131a) runs through the suspension layer (130), and having an upper hole (O1) and a lower hole (O2), a diameter of the upper hole (O1) is less than a diameter of the lower hole (O2), the flow body (M) flows into the suspension hole (131a) from the upper hole (O1), the protrusion structure (131b) is extended and disposed below the lower hole (O2), so that the flow body (M) is suspended under the suspension units (131) along the protrusion structure (131b).

2. The cell culture device (100) as claimed in claim 1, further comprising a separation layer (140), disposed between the diffusion layer (120) and the suspension layer (140), the separation layer (140) is provided with a plurality of separation holes (141) corresponding to the suspension units (131), the flow body (M) flows into the upper hole (O1) of the suspension hole (131a) through the flow channel (121) and the separation hole (141), wherein a diameter of the separation hole (141) is less than a diameter of the upper hole (O1), and the flow body (M) is a cell culture liquid.

3. The cell culture device (100) as claimed in claim 1, wherein a height of the protrusion structure (131b) is 1mm to 20 mm, and it is of a ring shape, extended and disposed below the lower hole (O2).

4. The cell culture device (100) as claimed in claim 1, wherein the top layer (110), the diffusion layer (120), and the suspension layer (130) formed integrally into a body.

5. The cell culture device (100) as claimed in claim 1, wherein the diameter of the upper hole (O1) is 1 mm to 15 mm, and the diameter of the lower hole (O2) is more than 1 mm to 30 mm.

6. The cell culture device (100) as claimed in claim 1, wherein thicknesses of the top layer (110), the diffusion layer (120), and the suspension layer (130) are 1 mm to 10 mm respectively.

7. An electromagnetic stimulation cell culturing system (S10) having a cell culture device (100), the cell culture device (100) is used to cultivate cells (C), comprising:
a top layer (110), having at least an inlet hole (111) and at least an outlet hole (112), the inlet hole (111) is used to input a flow body (M) into the cell culture device (100), the outlet hole (112) is used to output the flow body (M) from the cell culture device (100);
a diffusion layer (120), having at least a flow channel (121), the flow channel (121) corresponds to the inlet hole (111) and the outlet hole (112), and is used to guide the flow body (M);
a suspension layer (130), having a plurality of suspension units (131), the suspension units (131) correspond to the flow channel (121), the flow body (M) flows into the suspension units (131) through the flow channel (121), and is suspended under the suspension units (131), so that the cells (C) are cultivated in the flow body (M) under the suspension units (131), wherein, each of the suspension units (131) is provided with a suspension hole (131a) and a protrusion structure (131b), the suspension hole (131a) runs through the suspension layer (130), and having an upper hole (O1) and a lower hole (O2), a diameter of the upper hole (O1) is less than a diameter of the lower hole (O2), the flow body (M) flows into the suspension hole (131a) from the upper hole (O1), the protrusion structure (131b) is extended and disposed below the lower hole (O2), so that the flow body (M) is suspended under the suspension units (131) along the protrusion structure (131b);
a receiving plate (200), disposed below the cell culture device (100), and having a bottom seat (210) and a plurality of receiving slots (220), the plurality of receiving slots (220) correspond to the plurality of the suspension units (131) of the cell culture device (100) respectively, and is used to receive and collect the cells (C) and the flow body (M);
a flow body supply unit (400), connected to the cell culture device (100), and is used to supply the flow body (M) in circulation; and
an electromagnetic field generation unit (300), used to generate an electromagnetic field around the cell culture device (100).

8. The electromagnetic stimulation cell culturing system (S10) as claimed in claim 7 wherein the flow body supply unit (400) includes a pump (420) and a circulation pipe (410), the circulation pipe (410) is adapted to connect the inlet hole (111) to the outlet hole (112), and is used to input and output the flow body (M), and the pump (420) is adapted to push the flow body (M) to flow in circulation.

9. The electromagnetic stimulation cell culturing system (S10) as claimed in claim 7 wherein the flow body supply unit (400) includes a supply container (430) and a collection container (440), the supply container (430) is connected to the inlet hole (111) of the cell culture device (100) through the circulation pipe (410), and the collection container (440) is connected to the outlet hole (112) of the cell culture device (100) through the circulation pipe (410).

10. The electromagnetic stimulation cell culturing system (S10) as claimed in claim 7 wherein the electromagnetic field generation unit (300) includes at least a coil (310) and a magnetic field controller (320), the at least a coil (310) is used to generate the electromagnetic field, the magnetic field controller (320) is connected to the coil (310), and is used to control direction, frequency, and intensity of the electromagnetic field.

11. The electromagnetic stimulation cell culturing system as claimed in claim 7, further comprising: a power supplier (600) and a magnetic field detector (500), the power supplier (600) is used to supply power to the magnetic field controller (320), the magnetic field detector (500) is used to detect the electromagnetic field generated by the at least a coil (310).

## Patentansprüche

1. Zellkulturvorrichtung (100) zur Züchtung von Zellen, umfassend:
eine Deckschicht (110), welche mindestens eine Einlassöffnung (111) und mindestens eine Auslassöffnung (112) aufweist, wobei die Einlassöffnung (111) dazu dient, einen Fließkörper (M) in die Zellkulturvorrichtung (100) zuzuführen, wobei die Auslassöffnung (112) dazu dient, den Fließkörper (M) aus der Zellkulturvorrichtung (100) auszuführen,
eine Diffusionsschicht (120), die mindestens einen Strömungskanal (121) aufweist, wobei der Strömungskanal (121) der Einlassöffnung (111) und der Auslassöffnung (112) entspricht und dazu dient, den Fließkörper (M) zu führen, und eine Suspensionsschicht (130), mit einer Vielzahl von Suspensionseinheiten (131), wobei die Suspensionseinheiten (131) dem Strömungskanal (121) entsprechen, der Fließkörper (M) durch den Strömungskanal (121) in die Suspensionseinheiten (131) fließt und unter den Suspensionseinheiten (131) suspendiert ist, so dass die Zellen (C) in dem Fließkörper (M) jeweils unter den Suspensionseinheiten (131) kultiviert werden, wobei jede der Suspensionseinheiten (131) mit einer Suspensionsöffnung (131a) und einer Vorsprungsstruktur (131b) versehen ist, wobei die Suspensionsöffnung (131a) durch die Suspensionsschicht (130) verläuft und ein oberes Loch (O1) und ein unteres Loch (O2) aufweist, wobei ein Durchmesser des oberen Lochs (O1) kleiner ist als ein Durchmesser des unteren Lochs (O2), wobei der Fließkörper (M) von dem oberen Loch (O1) in die Suspensionsöffnung (131a) fließt, wobei die Vorsprungsstruktur (131b) verlängert und unterhalb des unteren Lochs (O2) angeordnet ist, so dass der Fließkörper (M) unter den Suspensionseinheiten (131) entlang der Vorsprungsstruktur (131b) suspendiert ist.

2. Zellkulturvorrichtung (100) nach Anspruch 1, ferner umfassend eine Trennschicht (140), die zwischen der Diffusionsschicht (120) und der Suspensionsschicht (130) angeordnet ist, wobei die Trennschicht (140) mit einer Vielzahl von Trennlöchern (141) versehen ist, die den Suspensionseinheiten (131) entsprechen, wobei der Fließkörper (M) in das obere Loch (O1) der Suspensionsöffnung (131a) durch den Strömungskanal (121) und das Trennloch (141) fließt, wobei ein Durchmesser des Trennlochs (141) kleiner als ein Durchmesser des oberen Lochs (O1) ist und der Fließkörper (M) eine Zellkulturflüssigkeit ist.

3. Zellkulturvorrichtung (100) nach Anspruch 1, bei welcher eine Höhe der Vorsprungsstruktur (131b) 1 mm bis 20 mm beträgt und eine Ringform aufweist, die sich unterhalb des unteren Lochs (O2) erstreckt und angeordnet ist.

4. Zellkulturvorrichtung (100) nach Anspruch 1, bei welcher die Deckschicht (110), die Diffusionsschicht (120) und die Suspensionsschicht (130) einstückig zu einem Körper ausgebildet sind.

5. Zellkulturvorrichtung (100) nach Anspruch 1, bei welcher der Durchmesser des oberen Lochs (O1) 1 mm bis 15 mm beträgt und der Durchmesser des unteren Lochs (O2) mehr als 1 mm bis 30 mm beträgt.

6. Zellkulturvorrichtung (100) nach Anspruch 1, bei welcher die Dicken der Deckschicht (110), der Diffusionsschicht (120) und der Suspensionsschicht (130) 1 mm bis 10 mm betragen.

7. Elektromagnetisch-stimuliertes Zellkultursystem (S10) mit einer Zellkulturvorrichtung (100), wobei die Zellkulturvorrichtung (100) zur Kultivierung von Zellen (C) verwendet wird, umfassend:
eine Deckschicht (110), die mindestens eine Einlassöffnung (111) und mindestens eine Auslassöffnung (112) aufweist, wobei die Einlassöffnung (111) dazu dient, einen Fließkörper (M) in die Zellkulturvorrichtung (100) zuzuführen, wobei die Auslassöffnung (112) dazu dient, den Fließkörper (M) aus der Zellkulturvorrichtung (100) auszuführen,
eine Diffusionsschicht (120), die mindestens einen Strömungskanal (121) aufweist, wobei der Strömungskanal (121) der Einlassöffnung (111) und der Auslassöffnung (112) entspricht und dazu dient, den Fließkörper (M) zu führen, eine Suspensionsschicht (130) mit einer Vielzahl von Suspensionseinheiten (131), wobei die Suspensionseinheiten (131) dem Strömungskanal (121) entsprechen, wobei der Fließkörper (M) durch den Strömungskanal (121) in die Suspensionseinheiten (131) fließt und unter den Suspensionseinheiten (131) suspendiert ist, so dass die Zellen (C) in dem Fließkörper (M) unter den Suspensionseinheiten (131) kultiviert werden, wobei jede der Suspensionseinheiten (131) mit einer Suspensionsöffnung (131a) und einer Vorsprungsstruktur (131b) versehen ist, wobei die Suspensionsöffnung (131a) durch die Suspensionsschicht (130) verläuft und ein oberes Loch (O1) und ein unteres Loch (O2) aufweist, wobei ein Durchmesser des oberen Lochs (O1) kleiner ist als ein Durchmesser des unteren Lochs (O2), wobei der Fließkörper (M) von dem oberen Loch (O1) in die Suspensionsöffnung (131a) fließt, wobei die Vorsprungsstruktur (131b) verlängert ist und unter dem unteren Loch (O2) angeordnet ist, so dass der Fließkörper (M) unter den Suspensionseinheiten (131) entlang der Vorsprungsstruktur (131b) suspendiert ist,
eine Aufnahmeplatte (200), die unterhalb der Zellkulturvorrichtung (100) angeordnet ist und einen Bodensitz (210) und eine Vielzahl von Aufnahmeschlitzen (220) aufweist, wobei die Vielzahl der Aufnahmeschlitze (220) jeweils der Vielzahl der Suspensionseinheiten (131) der Zellkulturvorrichtung (100) entspricht und verwendet wird, um die Zellen (C) und den Fließkörper (M) aufzunehmen und zu sammeln,
eine Fließkörper-Zuführeinheit (400), die mit der Zellkulturvorrichtung (100) verbunden ist und zur Zuführung des Fließkörpers (M) im Kreislauf verwendet wird, und
eine Einheit (300) zur Erzeugung eines elektromagnetischen Feldes, die verwendet wird, um ein elektromagnetisches Feld um die Zellkulturvorrichtung (100) zu erzeugen.

8. Elektromagnetisch-stimuliertes Zellkultursystem (S10) nach Anspruch 7, bei welchem die Fließkörper-Zuführeinheit (400) eine Pumpe (420) und ein Zirkulationsrohr (410) umfasst, wobei das Zirkulationsrohr (410) so angepasst ist, dass es die Einlassöffnung (111) mit der Auslassöffnung (112) verbindet, und verwendet wird, um den Fließkörper (M) einzugeben und auszugeben, und die Pumpe (420) so angepasst ist, dass sie den Fließkörper (M) zum Fließen im Kreislauf bringt.

9. Elektromagnetisch-stimuliertes Zellkultursystem (S10) nach Anspruch 7, bei welchem die Fließkörper-Zuführeinheit (400) einen Zuführbehälter (430) und einen Sammelbehälter (440) beinhaltet, wobei der Zuführbehälter (430) mit der Einlassöffnung (111) der Zellkulturvorrichtung (100) durch die Zirkulationsleitung (410) verbunden ist, und der Sammelbehälter (440) mit der Auslassöffnung (112) der Zellkulturvorrichtung (100) durch die Zirkulationsleitung (410) verbunden ist.

10. Elektromagnetisch-stimuliertes Zellkultursystem (S10) nach Anspruch 7, bei welchem die Einheit (300) zur Erzeugung eines elektromagnetischen Feldes mindestens eine Spule (310) und eine Magnetfeldsteuerung (320) umfasst, wobei die mindestens eine Spule (310) zur Erzeugung des elektromagnetischen Feldes verwendet wird, die Magnetfeldsteuerung (320) mit der Spule (310) verbunden ist und zur Steuerung von Richtung, Frequenz und Intensität des elektromagnetischen Feldes verwendet wird.

11. Elektromagnetisch-stimuliertes Zellkultursystem nach Anspruch 7, ferner umfassend: eine Stromversorgungseinrichtung (600) und einen Magnetfelddetektor (500), wobei die Stromversorgungseinrichtung (600) verwendet wird, um die Magnetfeldsteuerung (320) mit Strom zu versorgen, und der Magnetfelddetektor (500) verwendet wird, um das von der mindestens einen Spule (310) erzeugte elektromagnetische Feld zu erfassen.

## Revendications

1. Dispositif de culture cellulaire (100) utilisé pour cultiver des cellules, comprenant:
une couche supérieure (110), présentant au moins un trou d'entrée (111) et au moins un trou de sortie (112), le trou d'entrée (111) est utilisé pour entrer un corps d'écoulement (M) dans le dispositif de culture cellulaire (100), le trou de sortie (112) est utilisé pour sortir le corps d'écoulement (M) à partir du dispositif de culture cellulaire (100);
une couche de diffusion (120), présentant au moins un canal d'écoulement (121), le canal d'écoulement (121) correspond au trou d'entrée (111) et au trou de sortie (112), et est utilisé pour guider le corps d'écoulement (M); et
une couche de suspension (130), présentant une pluralité d'unités de suspension (131), les unités de suspension (131) correspondent au canal d'écoulement (121), le corps d'écoulement (M) s'écoule dans les unités de suspension (131) à travers le canal d'écoulement (121), et est suspendu sous les unités de suspension (131), de sorte que les cellules (C) sont cultivées dans le corps d'écoulement (M) sous les unités de suspension (131), respectivement, dans lequel chacune des unités de suspension (131) est pourvue d'un trou de suspension (131a) et d'une structure en saillie (131b), le trou de suspension (131a) s'étend à travers la couche de suspension (130), et présentant un trou supérieur (O1) et un trou inférieur (O2), un diamètre du trou supérieur (O1) est inférieur à un diamètre du trou inférieur (O2), le corps d'écoulement (M) s'écoule dans le trou de suspension (131a) à partir du trou supérieur (O1), la structure en saillie (131b) est étendue et disposée au-dessous du trou inférieur (O2), de sorte que le corps d'écoulement (M) est suspendu sous les unités de suspension (131) le long de la structure en saillie (131b).

2. Dispositif de culture cellulaire (100) selon la revendication 1, comprenant en outre une couche de séparation (140), disposée entre la couche de diffusion (120) et la couche de suspension (140), la couche de séparation (140) est pourvue d'une pluralité de trous de séparation (141) correspondant aux unités de suspension (131), le corps d'écoulement (M) s'écoule dans le trou supérieur (O1) du trou de suspension (131a) à travers le canal d'écoulement (121) et le trou de séparation (141), dans lequel un diamètre du trou de séparation (141) est inférieur à un diamètre du trou supérieur (O1), et le corps d'écoulement (M) est un liquide de culture cellulaire.

3. Dispositif de culture cellulaire (100) selon la revendication 1, dans lequel une hauteur de la structure en saillie (131b) est de 1 mm à 20 mm, et elle est d'une forme annulaire, étendue et disposée en dessous du trou inférieur (O2).

4. Dispositif de culture cellulaire (100) selon la revendication 1, dans lequel la couche supérieure (110), la couche de diffusion (120) et la couche de suspension (130) sont formées d'un seul tenant dans un corps.

5. Dispositif de culture cellulaire (100) selon la revendication 1, dans lequel le diamètre du trou supérieur (O1) est de 1 mm à 15 mm, et le diamètre du trou inférieur (O2) est de plus de 1 mm à 30 mm.

6. Dispositif de culture cellulaire (100) selon la revendication 1, dans lequel les épaisseurs de la couche supérieure (110), de la couche de diffusion (120) et de la couche de suspension (130) sont respectivement de 1 mm à 10 mm.

7. Système de culture cellulaire à stimulation électromagnétique (S10) présentant un dispositif de culture cellulaire (100), le dispositif de culture cellulaire (100) est utilisé pour cultiver des cellules (C), comprenant:
une couche supérieure (110), présentant au moins un trou d'entrée (111) et au moins un trou de sortie (112), le trou d'entrée (111) est utilisé pour entrer un corps d'écoulement (M) dans le dispositif de culture cellulaire (100), le trou de sortie (112) est utilisé pour sortir le corps d'écoulement (M) à partir du dispositif de culture cellulaire (100);
une couche de diffusion (120), présentant au moins un canal d'écoulement (121), le canal d'écoulement (121) correspond au trou d'entrée (111) et au trou de sortie (112), et est utilisé pour guider le corps d'écoulement (M);
une couche de suspension (130), présentant une pluralité d'unités de suspension (131), les unités de suspension (131) correspondent au canal d'écoulement (121), le corps d'écoulement (M) s'écoule dans les unités de suspension (131) à travers le canal d'écoulement (121), et est suspendu sous les unités de suspension (131), de sorte que les cellules (C) sont cultivées dans le corps d'écoulement (M) sous les unités de suspension (131), dans lequel chacune des unités de suspension (131) est pourvue d'un trou de suspension (131a) et d'une structure en saillie (131b), le trou de suspension (131a) s'étend à travers la couche de suspension (130), et présentant un trou supérieur (O1) et un trou inférieur (O2), un diamètre du trou supérieur (O1) est inférieur à un diamètre du trou inférieur (O2), le corps d'écoulement (M) s'écoule dans le trou de suspension (131a) à partir du trou supérieur (O1), la structure en saillie (131b) est étendue et disposée au-dessous du trou inférieur (O2), de sorte que le corps d'écoulement (M) est suspendu sous les unités de suspension (131) le long de la structure en saillie (131b);
une plaque de réception (200), disposée sous le dispositif de culture cellulaire (100), et présentant un siège inférieur (210) et une pluralité de fentes de réception (220), la pluralité de fentes de réception (220) correspond à la pluralité d'unités de suspension (131) du dispositif de culture cellulaire (100) respectivement, et est utilisée pour recevoir et collecter les cellules (C) et le corps d'écoulement (M);
une unité d'alimentation en corps d'écoulement (400), connectée au dispositif de culture cellulaire (100), et est utilisée pour alimenter le corps d'écoulement (M) en circulation; et
une unité de génération de champ électromagnétique (300), utilisée pour générer un champ électromagnétique autour du dispositif de culture cellulaire (100).

8. Système de culture cellulaire à stimulation électromagnétique (S10) selon la revendication 7, dans lequel l'unité d'alimentation en corps d'écoulement (400) inclut une pompe (420) et un tuyau de circulation (410), le tuyau de circulation (410) est adapté pour connecter le trou d'entrée (111) au trou de sortie (112), et est utilisé pour entrer et sortir le corps d'écoulement (M), et la pompe (420) est adaptée pour pousser le corps d'écoulement (M) à s'écouler en circulation.

9. Système de culture cellulaire à stimulation électromagnétique (S10) selon la revendication 7, dans lequel l'unité d'alimentation en corps d'écoulement (400) inclut un conteneur d'alimentation (430) et un conteneur de collecte (440), le conteneur d'alimentation (430) est connecté au trou d'entrée (111) du dispositif de culture cellulaire (100) via le tuyau de circulation (410), et le conteneur de collecte (440) est connecté au trou de sortie (112) du dispositif de culture cellulaire (100) via le tuyau de circulation (410).

10. Système de culture cellulaire à stimulation électromagnétique (S10) selon la revendication 7, dans lequel l'unité de génération de champ électromagnétique (300) inclut au moins une bobine (310) et un dispositif de commande de champ magnétique (320), la au moins une bobine (310) est utilisée pour générer le champ électromagnétique, le dispositif de commande de champ magnétique (320) est connecté à la bobine (310), et est utilisé pour commander une direction, une fréquence et une intensité du champ électromagnétique.

11. Système de culture cellulaire à stimulation électromagnétique selon la revendication 7, comprenant en outre : un dispositif d'alimentation en énergie (600) et un détecteur de champ magnétique (500), le dispositif d'alimentation en énergie (600) est utilisé pour alimenter en énergie le dispositif de commande de champ magnétique (320), le détecteur de champ magnétique (500) est utilisé pour détecter le champ électromagnétique généré par la au moins une bobine (310).
